(19) Europäisches Patentamt / European Patent Office / Office européen des brevets

(11) **EP 3 383 258 B1**

(12) **EUROPEAN PATENT SPECIFICATION**

(45) Date of publication and mention
of the grant of the patent:
**05.06.2019 Bulletin 2019/23**

(51) Int Cl.:
*A61B 5/0205* (2006.01)     *A61B 5/024* (2006.01)
*A61B 5/00* (2006.01)     *A61B 5/021* (2006.01)
*A61B 5/08* (2006.01)

(21) Application number: **16805781.8**

(22) Date of filing: **01.12.2016**

(86) International application number:
**PCT/EP2016/079390**

(87) International publication number:
**WO 2017/093379 (08.06.2017 Gazette 2017/23)**

(54) **DEVICE, SYSTEM AND METHOD FOR DETERMINING VITAL SIGN INFORMATION OF A SUBJECT**

VORRICHTUNG, SYSTEM UND VERFAHREN ZUR BESTIMMUNG DER
VITALZEICHENINFORMATIONEN EINES PATIENTEN

SYSTÈME ET PROCÉDÉ PERMETTANT DE DÉTERMINER LES INFORMATIONS DE SIGNES
VITAUX D'UNE PERSONNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.12.2015 EP 15197218**

(43) Date of publication of application:
**10.10.2018 Bulletin 2018/41**

(73) Proprietors:
• **Koninklijke Philips N.V.**
**5656 AE Eindhoven (NL)**
• **Technische Universiteit Eindhoven**
**5612 AZ Eindhoven (NL)**
Designated Contracting States:
**NL**

(72) Inventors:
• **DE HAAN, Gerard**
**5656 AE Eindhoven (NL)**
• **VAN GASTEL, Mark, Josephus, Henricus**
**5656 AE Eindhoven (NL)**

(74) Representative: **Ledeboer, Johannes Albertus
Philips Intellectual Property & Standards
High Tech Campus 5
5656 AE Eindhoven (NL)**

(56) References cited:
**US-A1- 2014 275 825     US-A1- 2015 104 088
US-A1- 2015 320 363**

• **FENG LITONG ET AL: "Motion-Resistant Remote
Imaging Photoplethysmography Based on the
Optical Properties of Skin", IEEE
TRANSACTIONS ON CIRCUITS AND SYSTEMS
FOR VIDEO TECHNOLOGY, IEEE SERVICE
CENTER, PISCATAWAY, NJ, US, vol. 25, no. 5, 1
May 2015 (2015-05-01), pages 879-891,
XP011580036, ISSN: 1051-8215, DOI:
10.1109/TCSVT.2014.2364415 [retrieved on
2015-05-01]**
• **DE HAAN G ET AL: "Improved motion robustness
of remote-PPG by using the blood volume pulse
signature", PHYSIOLOGICAL MEASUREMENT,
INSTITUTE OF PHYSICS PUBLISHING, BRISTOL,
GB, vol. 35, no. 9, 27 August 2014 (2014-08-27),
pages 1913-1926, XP020269523, ISSN: 0967-3334,
DOI: 10.1088/0967-3334/35/9/1913 [retrieved on
2014-08-27] cited in the application**

**Description**

FIELD OF THE INVENTION

The present invention relates to a device, system and method for determining vital sign information, in particular respiration information like the respiration rate or Traube-Hering-Mayer waves, of a subject, such as a person (e.g. a patient, elderly person, baby, etc.) or animal.

BACKGROUND OF THE INVENTION

Vital signs of a person, for example the heart rate (HR), the respiration rate (RR) or the arterial blood oxygen saturation, serve as indicators of the current state of a person and as powerful predictors of serious medical events. For this reason, vital signs are extensively monitored in inpatient and outpatient care settings, at home or in further health, leisure and fitness settings.

One way of measuring vital signs is plethysmography. Plethysmography generally refers to the measurement of volume changes of an organ or a body part and in particular to the detection of volume changes due to a cardiovascular pulse wave traveling through the body of a subject with every heartbeat.

Photoplethysmography (PPG) is an optical measurement technique that evaluates a time-variant change of light reflectance or transmission of an area or volume of interest. PPG is based on the principle that blood absorbs light more than surrounding tissue, so variations in blood volume with every heart beat affect transmission or reflectance correspondingly. Besides information about the heart rate, a PPG waveform can comprise information attributable to further physiological phenomena such as the respiration. By evaluating the transmittance and/or reflectivity at different wavelengths (typically red and infrared), the blood oxygen saturation can be determined.

Conventional pulse oximeters (also called contact PPG device herein) for measuring the heart rate and the (arterial) blood oxygen saturation (also called SpO2) of a subject are attached to the skin of the subject, for instance to a fingertip, earlobe or forehead. Therefore, they are referred to as 'contact' PPG devices. A typical pulse oximeter comprises a red LED and an infrared LED as light sources and one photodiode for detecting light that has been transmitted through patient tissue. Commercially available pulse oximeters quickly switch between measurements at a red and an infrared wavelength and thereby measure the transmittance of the same area or volume of tissue at two different wavelengths. This is referred to as time-division-multiplexing. The transmittance over time at each wavelength gives the PPG waveforms for red and infrared wavelengths. Although contact PPG is regarded as a basically non-invasive technique, contact PPG measurement is often experienced as being unpleasant and obtrusive, since the pulse oximeter is directly attached to the subject and any cables limit the freedom to move and might hinder a workflow. The same holds for contact sensors for respiration measurements.

Recently, non-contact, remote PPG (rPPG) devices (also called camera rPPG device herein) for unobtrusive measurements have been introduced. Remote PPG utilizes light sources or, in general radiation sources, disposed remotely from the subject of interest. Similarly, also a detector, e.g., a camera or a photo detector, can be disposed remotely from the subject of interest. Therefore, remote photoplethysmographic systems and devices are considered unobtrusive and well suited for medical as well as non-medical everyday applications. However, remote PPG devices typically achieve a lower signal-to-noise ratio.

Verkruysse et al., "Remote plethysmographic imaging using ambient light", Optics Express, 16(26), 22 December 2008, pp. 21434-21445 demonstrates that photoplethysmographic signals can be measured remotely using ambient light and a conventional consumer level video camera, using red, green and blue color channels.

Using PPG technology, vital signs can be measured, which are revealed by minute light absorption changes in the skin caused by the pulsating blood volume, i.e. by periodic color changes of the human skin induced by the blood volume pulse. As this signal is very small and hidden in much larger variations due to illumination changes and motion, there is a general interest in improving the fundamentally low signal-to-noise ratio (SNR). There still are demanding situations, with severe motion, challenging environmental illumination conditions, or high required accuracy of the application, where an improved robustness and accuracy of the vital sign measurement devices and methods is required, particularly for the more critical healthcare applications.

To achieve motion robustness, pulse-extraction methods profit from the color variations having an orientation in the normalized RGB color space which differs from the orientation of the most common distortions usually induced by motion. A known method for robust pulse signal extraction uses the known fixed orientation of the blood volume pulse in the normalized RGB color space to eliminate the distortion signals. Further background is disclosed in G. de Haan and A. van Leest, "Improved motion robustness of remote-PPG by using the blood volume pulse signature", Physiol. Meas. 35 1913, 2014, which describes that the different absorption spectra of arterial blood and bloodless skin cause the variations to occur along a very specific vector in a normalized RGB-space. The exact vector can be determined for a given light-spectrum and transfer-characteristics of the optical filters in the camera. It is shown that this "signature" can be used to design an rPPG algorithm with a much better motion robustness than the recent methods based on blind source separation, and even better than chrominance-based methods published earlier.

US 2014/0275825 A1 discloses a physiological monitoring system that may select a light signal for determining a physiological parameter. In some embodiments, the monitoring system may select a received light signal for further processing based on a physiological metric such as blood oxygen saturation value, or based on a system metric such as a signal-to-noise ratio. In some embodiments, the system may determine a light drive parameter based on a received signal. For example, the system may select a received light signal for further processing in order to determine a physiological parameter.

FENG LITONG ET AL: "Motion-Resistant Remote Imaging Photoplethysmography Based on the Optical Properties of Skin", IEEE TRANSACTIONS ON CIRCUITS AND SYSTEMS FOR VIDEO TECHNOLOGY, IEEE SERVICE CENTER, PISCATAWAY, NJ, US, vol. 25, no. 5, 1 May 2015 (2015-05-01), pages 879-891, XP011580036, discloses an optical Remote imaging photoplethysmography (RIPPG) signal model in which the origins of the RIPPG signal and motion artifacts can be clearly described. The region of interest (ROI) of the skin is regarded as a Lambertian radiator and the effect of ROI tracking is analyzed from the perspective of radiometry. By considering a digital color camera as a simple spectrometer, an adaptive color difference operation between the green and red channels to reduce motion artifacts is proposed. Based on the spectral characteristics of photoplethysmography signals, an adaptive bandpass filter is proposed to remove residual motion artifacts of RIPPG.

US 2015/0320363 A1 discloses a device for extracting physiological information indicative of at least one vital sign of a subject from detected electromagnetic radiation transmitted through or reflected from a subject comprises an input interface for receiving a data stream of detection data derived from detected electromagnetic radiation transmitted through or reflected from a skin region of a subject. The detection data comprises wavelength-dependent reflection or transmission information in at least two signal channels representative of respective wavelength portions. A signal mixer dynamically mixes the at least two signal channels into at least one mixed signal. A processor derives physiological information indicative of at least one vital sign from the at least one mixed signal, and a controller controls the signal mixer to limit the relative contributions of the at least two signal channels mixed into at least one mixed signal and/or the rate-of-change at which said relative contributions are allowed to dynamically change.

SUMMARY OF THE INVENTION

It is an object of the present invention to provide a device, system and a method for determining vital sign information of a subject, which provide an increased signal quality and an improved robustness of the obtained vital sign information with respect to motion and low SNR.

In a first aspect of the present invention, a device for determining vital sign information of a subject is presented, the device comprising:

- an input interface for obtaining at least two detection signals derived from detected electromagnetic radiation transmitted through or reflected from a skin region of a subject, wherein each detection signal comprises wavelength-dependent reflection or transmission information in a different wavelength channel,
- a first filter unit for filtering said at least two detection signals with a first filter to obtain at least two first bandwidth-limited detection signals,
- a weight computation unit for computing weights resulting, when applied in a weighted combination of said at least two first bandwidth-limited detection signals, in a first vital sign signal having reduced distortions,
- a vital sign signal computation unit for computing a second vital sign signal different from the first vital sign signal by using the computed weights and

    - either said first bandwidth-limited detection signals, if they include the frequency range of said second vital sign signal, to compute the first vital sign signal as a weighted combination of said at least two first bandwidth-limited detection signals and to filter the computed first vital sign signal with a second filter unit to obtain the second vital sign signal,
    - or a weighted combination of at least two second bandwidth-limited detection signals obtained by filtering said at least two detection signals with a further second filter unit, being differently bandwidth-limited than said first bandwidth-limited detection signals and including the frequency range of said second vital sign signal, and

- a vital sign determination unit for determining vital sign information from said second vital sign signal.

In a further aspect of the present invention, a system for determining vital sign information of a subject is presented, the system comprising:

- a detector for detecting electromagnetic radiation transmitted through or reflected from a skin region of a subject and for deriving at least two detection signals from the detected electromagnetic radiation, wherein each detection signal comprises wavelength-dependent reflection or transmission information in a different wavelength channel,
- a device as disclosed herein for determining respiration information from said derived at least two detection signals.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device and as defined in the dependent claims.

A PPG signal results from variations of the blood volume in the skin. Hence the variations give a characteristic pulsatility "signature" when viewed in different spectral components of the reflected/transmitted light. This signature is basically resulting as the contrast (difference) of the absorption spectra of the blood and that of the blood-less skin tissue. If the detector, e.g. a camera or sensor, has a discrete number of color channels, each sensing a particular part of the light spectrum, then the relative pulsatilities in these channels can be arranged in a "signature vector", also referred to as the "normalized blood-volume vector", Pbv. It has been shown in G. de Haan and A. van Leest, "Improved motion robustness of remote-PPG by using the blood volume pulse signature", Physiol. Meas. 35 1913, 2014, which is herein incorporated by reference, that if this signature vector is known then a motion-robust pulse signal extraction on the basis of the color channels and the signature vector is possible. For the quality of the pulse signal it is essential though that the signature is correct, as otherwise the known methods mixes noise into the output pulse signal in order to achieve the prescribed correlation of the pulse vector with the normalized color channels as indicated by the signature vector.

Details of the $P_{bv}$ method and the use of the normalized blood volume vector (called "predetermined index element having a set orientation indicative of a reference physiological information") have also been described in US 2013/271591 A1, which details are also herein incorporated by reference.

Although the above described known method (disclosed in G. de Haan and A. van Leest, "Improved motion robustness of remote-PPG by using the blood volume pulse signature", Physiol. Meas. 35 1913, 2014) using the known fixed orientation of the blood volume pulse in the normalized RGB color space to eliminate distortion signals could, in principle, be used to extract vital sign information (in particular respiration information represented by a respiration signal, such as the respiration rate) from detected electromagnetic radiation (or PPG signals derived therefrom) by just optimizing in a different frequency band, the performance suffers from the greater variability of both the period and the amplitude of the vital sign information signal. Also, since the frequency of the vital sign information signal (e.g. a respiration signal) is typically a factor of three lower than that of the pulse signal, much longer time intervals are required for optimizing the distortion suppression. These longer intervals can less quickly adapt to changing statistics of the distortions.

Hence, according to the present invention a different approach is proposed providing a much improved signal quality of the obtained vital sign information. This approach profits from the always available rather periodic color changes caused by cardiac activity to determine color variations that are orthogonal to motion artifacts and use those to detect the possibly irregular vital sign information signal, such as a respiration signal. The beating heart mainly causes pulsations in the arterial blood, while the pressure changes due to respiration act on the venous blood as well. Since the venous blood has a lower oxygenation level, with a somewhat higher absorption of red light the pulsation level in red is a bit higher for the respiration signal than it is for the pulse signal (also called first vital sign signal herein). However, it is sufficient to determine the orientation in a (pseudo-) color space that is orthogonal to motion artifacts and possible other distortions, using the pulse signal.

As long as this orthogonal direction does not line up with the direction of color changes due to respiration, a respiration signal can be observed in this direction. In this context, the weights given to the different detection signals (also called color channels herein) can be seen as a projection onto a line. An algorithm may be used to choose this line such that the projected distortions are minimized. Generally, the pulse and respiration signals will change the color into a different direction than the distortions and hence will not be minimized.

This also holds for Mayer waves which also lead to changing blood volumes. Although there may be slight difference in color orientation depending on the oxygenation levels of the varying blood volume, there is only a small chance that this direction coincides with the direction of motion-induced distortions (generally intensity variations or specular reflection changes lead to quite different color variations than blood volume variations).

Thus, the present invention is based on the idea to find the linear combination of the color channels (also called wavelength channels or frequency bands; colors are to be understood broadly here and may include wavelength channels in invisible parts of the spectrum), which suppresses the distortions best in a frequency band including the pulse rate, and consequently use this same linear combination to extract the desired vital sign information (e.g. represented by a vital sign information signal such as a respiration signal or Mayer waves) in a lower frequency band. Different options to find the weights of the linear combination are proposed and are the subject matter of preferred embodiments.

The detector of the proposed system may be configured in different ways, in particular to detect detection signals at different wavelengths, preferably depending on the kind of application and the system configuration. In preferred embodiment it is configured to derive detection signals at wavelengths around 650nm, 810nm and 900nm, or at wavelengths around 760nm, 800nm and 840nm, or at wavelengths around 475nm, 550nm and 650nm, or at wavelengths around 650nm and 800nm, or at wavelengths around 660nm, 760nm, 800nm and 840nm. Generally, each detection signal comprises wavelength-dependent reflection or transmission information in a different wavelength channel, which means that the different 'wavelength channels' have a different sensitivity for wavelengths. Hence, they can be sensitive for the same wavelengths, but then the relative sensitivities should be different. In other words, optical filters, which may be used for sensing, may be (partially) overlapping, but should be different.

In general, the at least two signal channels (detection signals) are selected from a wavelength interval between 300nm and 1000nm, in particular represent the wavelength portions corresponding to red, green and blue light. This is particularly used when the PPG signals are obtained from image signals acquired by a (e.g. conventional) video camera and when the above mentioned principles of remote PPG are used for deriving one or more vital signs. In other embodiments infrared light may also be used in addition or instead of another color channel. For instance, for night-time applications one or more infrared wavelengths may be used in addition or alternatively.

Generally, there exists a lot of freedom in choosing the wavelengths. It is advantageous if the wavelengths correspond to spectral regions where the blood absorption is very different, although there may be reasons that prevent the most logical choice here, like preference for invisible light, limitations of the sensor, availability of efficient light sources, etc.

Generally, the interaction of electromagnetic radiation, in particular light, with biological tissue is complex and includes the (optical) processes of (multiple) scattering, backscattering, absorption, transmission and (diffuse) reflection. The term "reflect" as used in the context of the present invention is not to be construed as limited to specular reflection but comprises the afore-mentioned types of interaction of electromagnetic radiation, in particular light, with tissue and any combinations thereof.

For obtaining a vital sign information signal of the subject the data signals of skin pixel areas within the skin area are evaluated. Here, a "skin pixel area" means an area comprising one skin pixel or a group of adjacent skin pixels, i.e. a data signal may be derived for a single pixel or a group of skin pixels.

The detector for detecting electromagnetic radiation transmitted through or reflected from a skin region of a subject and for deriving detection data from the detected electromagnetic radiation may be implemented in various ways. In one embodiment the detector comprises a plethysmography sensor configured for being mounted to a skin portion of the subject for acquiring photoplethysmography signals. Such a sensor may e.g. be an optical plethysmography sensor mounted to a finger or earlobe or a sensor arranged within a wristband or wristwatch.

In another embodiment the detector may comprise an imaging unit for acquiring a sequence of image frames of the subject over time, from which photoplethysmography signals can be derived using the principle of remote PPG. The data stream may thus comprise a sequence of image frames or, more precisely, a series of image frames comprising spectral information. For instance, RGB-images comprising color information can be utilized. However, also frames representing infrared and red information can form the sequence of frames. The image frames can represent the observed subject and further elements.

In an embodiment of the proposed device said first filter unit is configured to let at least the frequency range of a subject's pulse rate pass and suppress a DC component. The first filter unit may e.g. be configured, in particular for an adult subject, to let a frequency range pass having a lower limit in the range of 30-120 BPM (beats per minute), in particular 40-100 BPM, and an upper limit in the range of 100-240 BPM, in particular 180-220 BPM or, in particular for an infant or neonate subject, to let a frequency range pass having a lower limit in the range 50-140 BPM, in particular 70-120 BPM, and an upper limit in the range of 180-240 BPM, in particular 200-220 BPM. Since, as explained above, the strength of the PPG signal depends on a lot of parameters (skin-tone, temperature, body-part, etc.), the pulse signal and the desired vital sign information signal (e.g. a respiration signal) vary in strength. Since the respiration signal varies additionally with the breathing volume and chest/abdominal breathing, the strength of the pulse signal is used to calibrate the amplitude of the respiration signal, assuming the pulse amplitude to be relatively stable apart from the parameters mentioned above.

In another embodiment the first filter unit is configured to additionally let the frequency range of a subject's respiration signal and/or Mayer waves pass. The first filter unit may e.g. be configured, in particular for an adult subject, to let a frequency range pass having a lower limit in the range of 5-25 BPM, in particular 10-20 BPM, and an upper limit in the range of 100-240 BPM, in particular 180-220 BPM or, for an infant or neonate subject, to let a frequency range pass having a lower limit in the range 5-25 BPM, in particular 10-20 BPM, and an upper limit in the range of 180-240 BPM, in particular 200-220 BPM. This allows different ways to calculate the second vital sign signal, e.g. avoids the use of a second filter unit, as will be explained below in detail.

In another embodiment said weight computation unit is configured to compute the weights such that

- the weighted combination has a covariance with the individual detection signals that corresponds, as closely as possible, to a predefined vector,
- intensity variations and specular reflections are suppressed, or
- a demixing matrix is computed to identify independent signals in the detection channels and a vital sign signal is chosen from the independent signals using a second criterion.

Thus, alternative methods as e.g. disclosed in the above cited paper G. de Haan and A. van Leest, "Improved motion robustness of remote-PPG by using the blood volume pulse signature" may be used to compute the weights.

As mentioned, the computation of the second vital sign signal may be performed in different ways. According to one option, the vital sign signal computation unit may be configured to compute the second vital sign signal by a weighted combination of the at least two second bandwidth-limited detection signals using the computed weights. Hereby, the at least two second bandwidth-limited detection signals may be obtained by use of a second filter unit for filtering said at least two detection signals with a second filter. In this context it shall be noted that the weighting and the second filtering may be done in reversed order.

According to an alternative option said vital sign signal computation unit is configured to compute a first vital sign signal by a weighted combination of said at least two first bandwidth-limited detection signals using the computed weights and wherein the device further comprises a second filter unit for filtering said first vital sign signal with a second filter to obtain said second vital sign signal.

In both options, the weights are computed using signals that include the pulse, and they may be applied to signals that do not include the pulse, or, if the second vital sign signal is in a sub-band of the first vital sign signal, the weights have already been applied to obtain the combined signal so that the second vital sign signal is obtained by re-filtering the first sign signal.

Further, in both options the order of weighing and the first filtering is, in general, arbitrary. However, since the weights are computed from the first bandwidth limited detection signals it is preferable to first apply the first filter and then determine the weights from the filtered detection signals, rather than applying the weights to the unfiltered detection signals and filter the weighted result thereafter.

The respiration signal and the pulse signal do not cause exactly the same color variation, since the pulse occurs in the arterial (oxygenated) blood only, while the respiration signal also occurs in the venous blood which has a different absorption. By limiting the first filter to include the pulse but exclude the respiration, the weights can be better optimized to be orthogonal to the motion-induced distortions.

The second filter unit is preferably configured to let at least the frequency range of a subject's respiration signal and/or Traube-Hering-Mayer waves, i.e. the frequency range of the desired vital sign information, pass and suppress at least the frequency range of a subject's pulse signal. The second filter unit is particularly configured to let a frequency range pass having a lower limit in the range of 5-25 BPM, in particular 10-20 BPM, and an upper limit in the range of 25-70 BPM, in particular 30-60 BPM.

In still another option said vital sign signal computation unit is configured to compute a first vital sign signal by a weighted combination of said at least two first bandwidth-limited detection signals using the computed weights, and the device further comprises a characteristics detector for detection of a characteristic of said first vital sign signal, in particular for peak detection in a frequency domain representation and/or amplitude or standard deviation detection in a time domain representation of said first vital sign signal, to obtain a gain and a multiplication unit for multiplying the second vital sign signal with said gain. The pulse signal (first vital sign signal) resulting from the weights and first bandwidth-limited detection signals are used to compute a gain of the second vital sign signal. The gain essentially stabilizes the amplitude of the pulse signal, i.e. it is proportional to the inverse of the amplitude of the pulse signal. By computing the gain that stabilizes the pulse amplitude (the inverse of the pulse amplitude measured in the time or in the frequency domain), this gain can e.g. be applied to the respiration signal so that it also has a stable amplitude (since the same weights are used). In other words, the second vital sign signal may be adapted to the amplitude / standard deviation of the first vital sign signal, or to a detected peak height, in particular the RMS-value of a detected peak and a predetermined frequency range around the detected peak in the spectrum of the first vital sign signal. For adjusting the amplitude of the second vital sign signal, the amplitude or RMS-value in a small band around the fundamental frequency of the pulse signal is used to determine the gain of the second vital sign signal.

The characteristics detector may hereby be configured to limit the characteristics detection to a frequency range of a subject's pulse signal. The first vital sign signal may contain still different frequencies. In the frequency domain implementation, it is possible to do a peak detection to find the likely pulse rate and consequently measure the RMS-value (which corresponds to the amplitude in the time domain) of the actual pulse signal.

In another embodiment the device is configured to compute a number of second vital sign signals, each from a different set of at least two detection signals derived from detected electromagnetic radiation transmitted through or reflected from different skin regions of the subject, and wherein said respiration determination unit is configured to determine the respiration information from a combination of said second vital sign signals. This provides improved accuracy and reliability of the determined vital sign information by combining parallel measurements at different sub-regions (spatial redundancy).

In another embodiment said input interface is configured to obtain different sets of at least two detection signals derived from detected electromagnetic radiation transmitted through or reflected from different skin regions of the subject, wherein said weight computation unit is configured to compute weights per set of at least two detection signals, wherein said vital sign signal computation unit is configured to compute, per set of at least two detection signals, a first preliminary vital sign signal by a weighted combination of said at least two first bandwidth-limited detection signals using the computed weights of the respective set of at least two detection signals and to compute said first vital sign signal by combining said first preliminary vital sign signals computed for the different sets of at least two detection signals. Preferably, the device further comprises a second filter unit for filtering said first vital sign signal with a second filter to obtain said second vital sign signal. Also in this embodiment the order of weighing and the first filtering is, in general, arbitrary. Further, the first filter may be configured to let frequencies pass including or excluding the frequencies of the desired vital sign information, in particular the frequencies of respiration information.

Still further, in an embodiment said weight computation unit is configured to compute said weights by setting a gain, used in the computation, such that the amplitude of said first vital sign signal or of the standard deviation of said first vital sign signal or of a characteristic, in particular a peak or a RMS-value of a small frequency range (around a peak), in the frequency domain representation of said first vital sign signal is constant over time.

BRIEF DESCRIPTION OF THE DRAWINGS

These and other aspects of the invention will be apparent from and elucidated with reference to the embodiments described hereinafter. In the following drawings:

Fig. 1 shows a schematic diagram of a system according to the present invention,
Fig. 2 shows a diagram of the absorption spectrum of oxygenated and non-oxygenated blood,
Fig. 3 shows a schematic diagram of a first embodiment of a device according to the present invention,
Fig. 4 shows a schematic diagram of a second embodiment of a device according to the present invention, and
Fig. 5 shows a schematic diagram of a third embodiment of a device according to the present invention.

DETAILED DESCRIPTION OF THE INVENTION

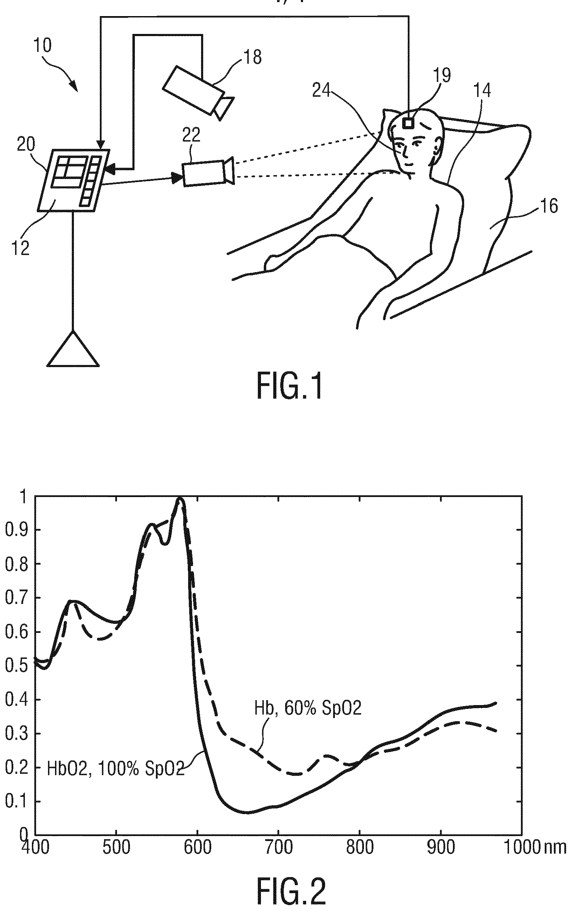

Fig. 1 shows a schematic diagram of a system 10 according to the present invention including a device 12 for determining a vital sign information (in particular a vital sign information signal) of a subject 14 from detected electromagnetic radiation transmitted through or reflected from a subject. The subject 14, in this example a patient, lies in a bed 16, e.g. in a hospital or other healthcare facility, but may also be a neonate or premature infant, e.g. lying in an incubator, or person at home or in a different environment, such as an athlete doing sports.

For the following explanation, the vital sign information to be determined shall be respiration information, such as the respiration rate, which is preferably represented by a respiration signal. Further respiration information may include the waveform, the intervals between exhale and inhale, the amplitude, and/or the variability of the respiratory rate. However, the invention may also be applied for determining Traube-Hering-Mayer waves (also called Mayer waves or THM waves), in which case the bandwidth of signals and/or filters may be different since THM waves are around 6 BMP, which will also be mentioned below.

There exist different embodiments for a detector for detecting electromagnetic radiation transmitted through or reflected from a subject, which may alternatively (which is preferred) or together be used. In the embodiment of the system 10 two different embodiments of the detector are shown and will be explained below. Both embodiments of the detector are configured for deriving at least two detection signals from the detected electromagnetic radiation, wherein each detection signal comprises wavelength-dependent reflection or transmission information in a different wavelength channel. Herby, optical filters used are preferably different, but can be overlapping. It is sufficient if their wavelength-dependent transmission is different.

In one embodiment the detector comprises a camera 18 (also referred to as imaging unit, or as camera-based or remote PPG sensor) including a suitable photosensor for (remotely and unobtrusively) capturing image frames of the subject 14, in particular for acquiring a sequence of image frames of the subject 14 over time, from which photoplethysmography signals can be derived. The image frames captured by the camera 18 may particularly correspond to a video sequence captured by means of an analog or digital photosensor, e.g. in a (digital) camera. Such a camera 18 usually includes a photosensor, such as a CMOS or CCD sensor, which may also operate in a specific spectral range (visible, IR) or provide information for different spectral ranges. The camera 18 may provide an analog or digital signal. The image frames include a plurality of image pixels having associated pixel values. Particularly, the image frames include pixels representing light intensity values captured with different photosensitive elements of a photosensor. These photosensitive elements may be sensitive in a specific spectral range (i.e. representing a specific color). The image frames include at least some image pixels being representative of a skin portion of the subject. Thereby, an image pixel may correspond to one photosensitive element of a photodetector and its (analog or digital) output or may be determined based on a combination (e.g. through binning) of a plurality of the photosensitive elements.

In another embodiment the detector comprises one or more optical photoplethysmography sensor(s) 19 (also referred to as contact PPG sensor(s)) configured for being mounted to a skin portion of the subject 14 for acquiring photoplethysmography signals. The PPG sensor(s) 19 may e.g. be designed in the form of a patch attached to a subject's forehead for measuring the blood oxygen saturation or a heart rate sensor for measuring the heart rate, just to name a few of all the possible embodiments.

When using a camera 18 the system 10 may further optionally comprise a light source 22 (also called illumination source), such as a lamp, for illuminating a region of interest 24, such as the skin of the patient's face (e.g. part of the cheek or forehead), with light, for instance in a predetermined wavelength range or ranges (e.g. in the red, green and/or infrared wavelength range(s)). The light reflected from said region of interest 24 in response to said illumination is detected by the camera 18. In another embodiment no dedicated light source is provided, but ambient light is used for illumination of the subject 14. From the reflected light only light in a desired wavelength ranges (e.g. green and red or infrared light, or light in a sufficiently large wavelength range covering at least two wavelength channels) may be detected and/or evaluated.

The device 12 is further connected to an interface 20 for displaying the determined information and/or for providing medical personnel with an interface to change settings of the device 12, the camera 18, the PPG sensor(s) 19, the light source 22 and/or any other parameter of the system 10. Such an interface 20 may comprise different displays, buttons, touchscreens, keyboards or other human machine interface means.

A system 10 as illustrated in Fig. 1 may, e.g., be located in a hospital, healthcare facility, elderly care facility or the like. Apart from the monitoring of patients, the present invention may also be applied in other fields such as neonate monitoring, general surveillance applications, security monitoring or so-called live style environments, such as fitness equipment, a wearable, a handheld device like a smartphone, or the like. The uni- or bidirectional communication between the device 12, the camera 18, the PPG sensor(s) 19 and the interface 20 may work via a wireless or wired communication interface. Other embodiments of the present invention may include a device 12, which is not provided stand-alone, but integrated into the camera 18 or the interface 20.

There exist several known methods to obtain a pulse signal S from (normalized) detection signals $C_n$, said methods being referred to as ICA, PCA, $P_{BV}$, CHROM, and ICA/PCA guided by $P_{BV}$/CHROM, which have also been described in the above cited paper of de Haan and van Leest. These methods can be interpreted as providing the pulse signal S as a mixture of different wavelength channels, e.g. red, green and blue signals from a color video camera, but they differ in the way to determine the optimal weighting scheme. In these methods the resulting weights are aimed at a mixture in which the distortions disappear, i.e. the "weighting vector" is substantially orthogonal to the main distortions usually caused by subject motion and/or illumination variations.

In the following some basic considerations with respect to the $P_{bv}$ method shall be briefly explained.

The beating of the heart causes pressure variations in the arteries as the heart pumps blood against the resistance of the vascular bed. Since the arteries are elastic, their diameter changes in sync with the pressure variations. These diameter changes occur even in the smaller vessels of the skin, where the blood volume variations cause a changing absorption of the light.

The unit length normalized blood volume pulse vector (also called signature vector) is defined as $P_{bv}$, providing the relative PPG-strength in the red, green and blue camera signal. To quantify the expectations, the responses $H_{red}(w)$, $H_{green}(w)$ and $H_{blue}(w)$ of the red, green and blue channel, respectively, were measured as a function of the wavelength w, of a global-shutter color CCD camera1, the skin reflectance of a subject, $\rho_s(w)$, and used an absolute PPG-amplitude curve PPG(w). From these curves, shown e.g. in Fig. 2 of the above cited paper of de Haan and van Leest, the blood volume pulse vector $P_{bv}$ is computed as:

$$\vec{P}_{\text{bv}}^T = \begin{bmatrix} \dfrac{\int\limits_{w=400}^{700} H_{\text{red}}(w)I(w)PPG(w)\,dw}{\int\limits_{w=400}^{700} H_{\text{red}}(w)I(w)\rho_s(w)\,dw} \\ \dfrac{\int\limits_{w=400}^{700} H_{\text{green}}(w)I(w)PPG(w)\,dw}{\int\limits_{w=400}^{700} H_{\text{green}}(w)I(w)\rho_s(w)\,dw} \\ \dfrac{\int\limits_{w=400}^{700} H_{\text{blue}}(w)I(w)PPG(w)\,dw}{\int\limits_{w=400}^{700} H_{\text{blue}}(w)I(w)\rho_s(w)\,dw} \end{bmatrix}$$

which, using a white, halogen illumination spectrum I(w), leads to a normalized $P_{bv}$ = [0.27, 0.80, 0.54]. When using a more noisy curve the result may be $P_{bv}$ = [0.29, 0.81, 0.50].

The blood volume pulse predicted by the used model corresponds reasonably well to an experimentally measured normalized blood volume pulse vector, $P_{bv}$ = [0.33, 0.77, 0.53] found after averaging measurements on a number of subjects under white illumination conditions. Given this result, it was concluded that the observed PPG-amplitude, particularly in the red, and to a smaller extent in the blue camera channel, can be largely explained by the crosstalk from wavelengths in the interval between 500 and 600 nm. The precise blood volume pulse vector depends on the color filters of the camera, the spectrum of the light and the skin-reflectance, as the model shows. In practice the vector turns out to be remarkably stable though given a set of wavelength channels (the vector will be different in the infrared compared to RGB-based vector).

It has further been found that the relative reflectance of the skin, in the red, green and blue channel under white illumination does not depend much on the skin-type. This is likely because the absorption spectra of the blood-free skin is dominated by the melanin absorption. Although a higher melanin concentration can increase the absolute absorption considerably, the relative absorption in the different wavelengths remains the same. This implies an increase of melanin darkens the skin, but hardly changes the normalized color of the skin. Consequently, also the normalized blood volume pulse $P_{bv}$ is quite stable under white illumination. In the infrared wavelengths the influence of melanin is further reduced as its maximum absorption occurs for short wavelengths (UV-light) and decreases for longer wavelengths.

The stable character of $P_{bv}$ can be used to distinguish color variations caused by blood volume change from variations due to alternative causes. The resulting pulse signal S using known methods can be written as a linear combination (representing one of several possible ways of "mixing") of the individual DC-free normalized color channels:

$$S = W\,C_n$$

with $WW^T = 1$ and where each of the three rows of the 3 x N matrix $C_n$ contains N samples of the DC-free normalized red, green and blue channel signals $R_n$, $G_n$ and $B_n$, respectively, i.e.:

$$\vec{R}_n = \frac{1}{\mu(\vec{R})}\vec{R} - 1, \quad \vec{G}_n = \frac{1}{\mu(\vec{G})}\vec{G} - 1, \quad \vec{B}_n = \frac{1}{\mu(\vec{B})}\vec{B} - 1.$$

Here the operator $\mu$ corresponds to the mean. Key difference between the different methods is in the calculation of the weighting vector W. In one method, the noise and the PPG signal may be separated into two independent signals built as a linear combination of two color channels. One combination approximated a clean PPG signal, the other contained noise due to motion. As an optimization criterion the energy in the pulse signal may be minimized. In another method a linear combination of the three color channels may be used to obtain the pulse signal. In still further methods, the ICA or the PCA may be used to find this linear combination. Since it is a priori unknown which weighted color signal is the pulse signal all of them used the periodic nature of the pulse signal as the selection criterion.

The $P_{bv}$ method generally obtains the mixing coefficients using the blood volume pulse vector as basically described in US 2013/271591 A1 and the above cited paper of de Haan and van Leest. The best results are obtained if the band-passed filtered versions of $R_n$, $G_n$ and $B_n$ are used. According to this method the known direction of $P_{bv}$ is used to discriminate between the pulse signal and distortions. This not only removes the assumption (of earlier methods) that the pulse is the only periodic component in the video, but also eliminates assumptions on the orientation of the distortion signals. To this end, it is assumed as before that the pulse signal is built as a linear combination of normalized color signals. Since it is known that the relative amplitude of the pulse signal in the red, green and blue channel is given by $P_{bv}$, the weights, $W_{PBV}$, are searched that give a pulse signal S, for which the correlation with the color channels $R_n$, $G_n$, and $B_n$ equals $P_{bv}$

$$\vec{S}C_n^T = k\vec{P}_{bv} \Leftrightarrow \vec{W}_{PBV}C_nC_n^T = k\vec{P}_{bv}, \qquad (1)$$

and consequently the weights determining the mixing are determined by

$$\vec{W}_{PBV} = k\vec{P}_{bv}Q^{-1} \text{ with } Q = C_nC_n^T, \qquad (2)$$

and the scalar k is determined such that $W_{PBV}$ has unit length. It is concluded that the characteristic wavelength dependency of the PPG signal, as reflected in the normalized blood volume pulse, $P_{bv}$, can be used to estimate the pulse signal from the time-sequential RGB pixel data averaged over the skin area. This algorithm is referred to as the $P_{bv}$ method.

Hence, as explained above, a pulse signal results as a weighted sum of the at least two detection signals $C_n$. Since all detection signals $C_n$ contain the pulse and different levels of (common) noise, the weighting (of the detection signals to obtain the pulse signal) can lead to a pure noise-free pulse. This is why ICA and PCA can be used to separate noise and pulse. According to the present invention this is done differently.

Fig. 2 shows a diagram of the absorption spectra of blood for oxygenated blood (SpO2=100%) and non-oxygenated blood (SpO2=60%). As can be seen, the absorption spectrum of blood depends on the oxygen saturation, particularly in the wavelengths around 650nm. This causes the respiration to induce a slightly stronger absorption change in the red wavelength range. It is clear from Fig. 2 though that the absorption in the green wavelength range (around 550nm) and blue wavelength range (around 450nm) is much higher.

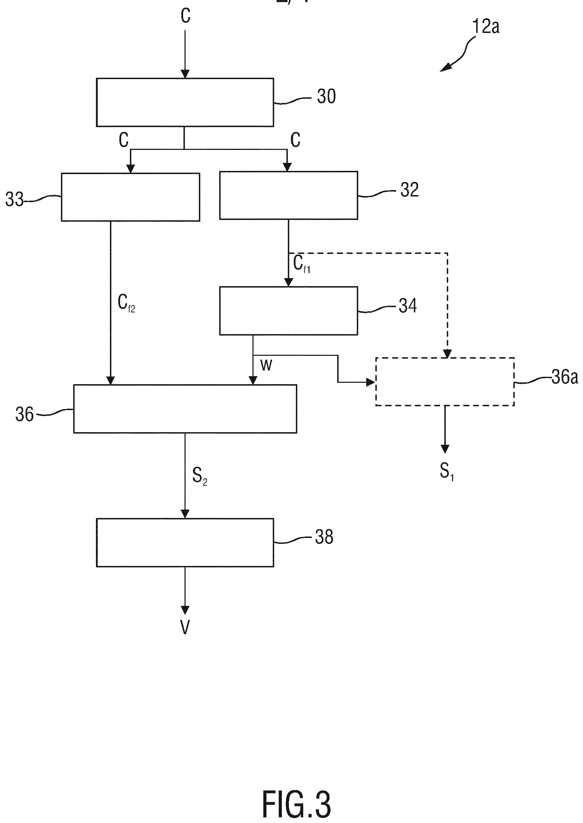

Fig. 3 shows a schematic illustration of a first embodiment 12a of the device 12 according to the present invention. The device 12a comprises an input interface 30 for obtaining at least two detection signals C derived from detected electromagnetic radiation transmitted through or reflected from a skin region of the subject 14. The data stream of detection data, i.e. the detection signals C, is e.g. provided by the camera 18 and/or one or more PPG sensor(s) 19, wherein each detection signal comprises wavelength-dependent reflection or transmission information in a different wavelength channel.

A first filter unit 32 filter said at least two detection signals C with a first filter to obtain at least two first bandwidth-limited detection signals $C_{f1}$.

A weight computation unit 34 computes weights w resulting, when applied in a weighted combination of said at least two first bandwidth-limited detection signals $C_{f1}$, in a first vital sign signal $S_1$ having reduced distortions. The first vital sign signal $S_1$ is thereby not necessarily determined, but only the weights w are actually determined. The first vital sign signals is only determined in certain embodiments.

In parallel, a second filter unit 33 filters said at least two detection signals C with a second filter to obtain at least two second bandwidth-limited detection signals $C_{f2}$. Hereby, the second filter is configured such that the second bandwidth-limited detection signals $C_{f2}$ are differently bandwidth-limited than said first bandwidth-limited detection signals $C_{f1}$ and particularly include the frequency range of said vital sign information (e.g. of a respiration signal and/or Mayer waves).

A vital sign signal computation unit 36 computes a second vital sign signal $S_2$ using the computed weights w and said at least two second bandwidth-limited detection signals $C_{f2}$. The second vital sign signal $S_2$ is hereby preferably computed by a weighted combination of the at least two second bandwidth-limited detection signals $C_{f2}$ using the computed weights w.

Hereby, "differently bandwidth-limited" means that it includes different frequencies or different frequency ranges. For instance, the first bandwidth-limited signals may include only the frequency range of pulse frequencies or additionally of respiration frequencies, and the second bandwidth-limited signals may include only the frequency range of respiration frequencies.

A vital sign determination unit 38 finally determines vital sign information V from said second vital sign signal $S_2$.

In a first embodiment, in line with Eq. (10) of the above cited paper of G. de Haan and A. van Leest, "Improved motion robustness of remote-PPG by using the blood volume pulse signature" and using (almost) the same notation, s weight vector $W_{PBV}$ can be found according to:

$$W_{PBV} = kP_{bv}Q^{-1} \text{ with } Q = C_{fr+fp}C_{fr+fp}^{T} \qquad (1)$$

where $C_{fr+fp}$ contains the signals of the DC-free, normalized and filtered three color channels of a camera and represents the first bandwidth-limited detection signals $C_{f1}$ in this embodiment. An optional good choice for $P_{bv}$ = [0.33, 0.77, 0.53], but other choices (e.g. as described above) are possible as well. This first filter is designed to include the range of pulse rates, e.g. 100-180 BPM for a neonate, or 40-220BPM for an adult subject. Preferably, the range also includes the range of respiratory frequencies to make sure that also low frequency distortions are eliminated as much as possible (indicated by the notation $C_{fr+fp}$). This may lead to a filter design passing frequencies in a range from 10-200 BPM.

Applying this weighting vector to $C_{fr+fp}$ gives a first vital sign signal $S_1$, which carries both the pulse signal and the respiration signal (and/or possibly Mayer waves):

$$S_1 = W_{PBV}C_{fr+fp} \qquad (2)$$

Generally, the calculation of the first vital sign signal $S_1$ is not mandatory, as represented by the schematic diagram shown in Fig. 3. Optionally, however, the first vital sign signal $S_1$ may also be determined by the vital sign signal computation unit, represented in broken lines by the unit 36a is Fig. 3, as a weighted combination of said at least two first bandwidth-limited detection signals $C_{f1}$ using the computed weights w.

A second vital sign signal $S_2$ representing the respiration signal is consequently calculated as:

$$S_2 = W_{PBV}C_{fr} \qquad (3)$$

where $C_{fr}$ contains the differently filtered DC-free normalized color channels and represents the second bandwidth-limited detection signals $C_{f2}$ in this embodiment. The pass-band of this second filter only includes the expected respiratory frequencies, e.g. between 8 and 30 BPM for an adult, or 20-60 BPM for a neonate.

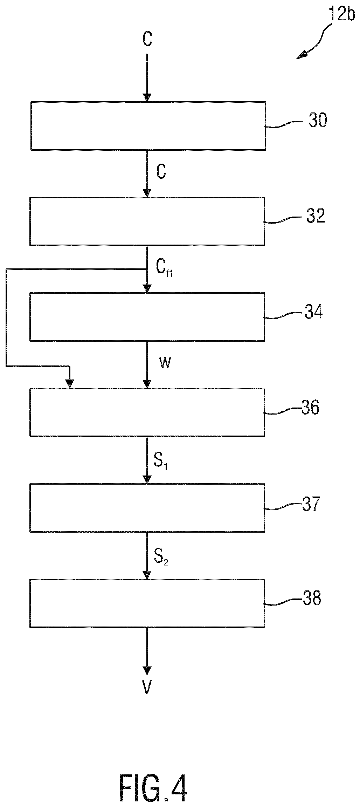

Fig. 4 shows a schematic illustration of a second embodiment 12b of the device 12 according to the present invention. According to this embodiment the vital sign signal computation unit 38 is configured to compute the second vital sign signal $S_2$ using the computed weights w and said first bandwidth-limited detection signals $C_{f1}$, which, in this embodiment, preferably include the frequency range of said second vital sign signal in addition to the frequency range of the pulse signal. The computation of the second vital sign signal $S_2$ is particularly performed in two steps. In a first step the vital sign signal computation unit 36 computes a first vital sign signal $S_1$ by a weighted combination of said at least two first bandwidth-limited detection signals $C_{f1}$ using the computed weights w. Further, in a second step, a second filter unit 37 (which may be part of the vital sign signal computation unit 36) filters said first vital sign signal $S_1$ with a second filter to obtain said second vital sign signal $S_2$.

Hence, if the first filter had a pass-band that included both pulse rates and respiration rates, the second vital sign signal $S_2$ can be obtained by re-filtering the first vital sign signal $S_1$ with the second filter. The second filter unit 37 is preferably configured to let at least the frequency range of a subject's respiration signal and/or Mayer waves pass and suppress at least the frequency range of a subject's pulse signal, in particular to let a frequency range pass having a lower limit in the range of 5-25 BPM, in particular 10-20 BPM, and an upper limit in the range of 25-70 BPM, in particular 30-60 BPM.

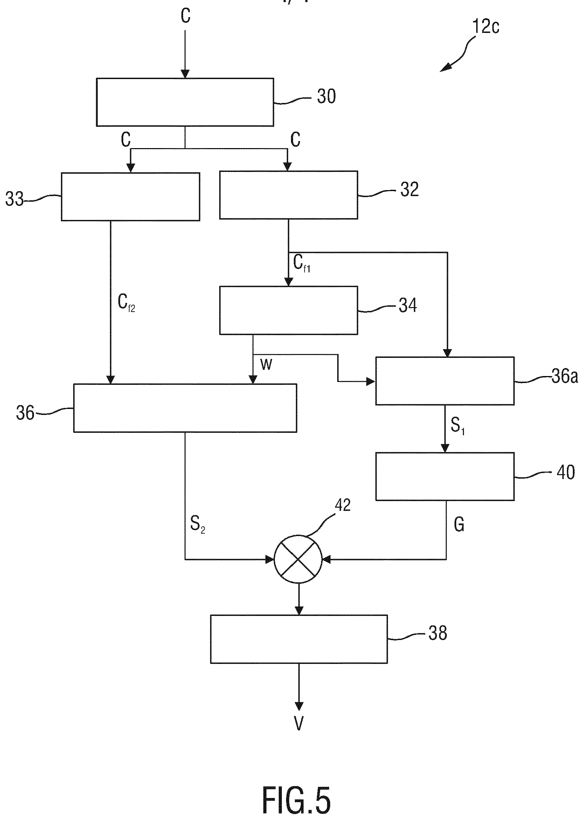

Fig. 5 shows a schematic illustration of a third embodiment 12c of the device 12 according to the present invention. According to this embodiment the first vital sign signal $S_1$ and the second vital sign signal $S_2$ are computed as illustrated above in the first (or second) embodiment. A peak detector 40 is provided for peak detection to determine the amplitude of first vital sign signal $S_1$. This may be done in the time domain by computing the amplitude/standard deviation of the first vital sign signal $S_1$, possibly after bandpass-filtering it to prevent influence of noise, or in the frequency domain by computing the RMS-value of the frequency bins around the pulse rate. The idea hereby is to use the amplitude of the pulse to set the gain G for the second vital sign signal $S_2$ (e.g. the respiration signal). Using the amplitude of the pulse (i.e. the first vital sign signal $S_1$) the inverse of this amplitude is used to normalize the amplitude of the second vital sign signal $S_2$ by multiplication of the second vital sign signal $S_2$ with the gain G in a multiplication unit 42 to obtain a normalized second vital sign signal $S_2$, from which the desired vital sign information V can then be derived.

Of course a multiplication with a constant gain in the multiplication unit 42 is further allowed, i.e. the resulting gain should be inversely proportional to the amplitude of the first vital sign signal $S_1$.

The peak detector may be particularly configured to limit the peak detection to a frequency range of a subject's pulse signal. Hence, the strength of the pulse signal is used to determine the gain needed to show the desired vital sign information signal with a substantially constant relative amplitude.

Thus, according to this embodiment peak detection may be performed in the Fourier domain of the first vital sign signal, limiting the frequency range, for peak detection, to the pulse frequencies. The second vital sign is then obtained as described above, but its amplitude is modified with a gain factor.

In a preferred embodiment, the above described processing uses an overlap-add-process, as described in the above cited paper of G. de Haan and A. van Leest, "Improved motion robustness of remote-PPG by using the blood volume pulse signature", where at least the optimization of equation (1) is performed on short intervals, typically a few seconds, to allow for distortion elimination even with changing statistics of the distortions over time. Also the above described synthesizing of the second vital sign signal from the first vital sign signal is performed on each interval. In case of filtering to obtain the second vital sign signal, this can also be performed after the overlap-add procedure.

In order to keep the amplitude of the respiration signal meaningful regardless the distortions (which affect the weights) and the strength of the breathing, the time-varying (fixed on each overlap-add interval) gain, k, of the output signal, which is included in the weighting vector $W_{PBV}$ (see equation (1)), can be selected such that the pulse signal in the first vital sign signal has a constant amplitude, e.g. by dividing the signals by the standard deviation of the first signal in the frequency band of the pulse signal. As a possible implementation, third band-width limited detection signals $C_{fp}$, i.e. the DC-free, normalized color channels filtered with a third band-pass filter, selecting the pulse rate frequency range only and choosing the gain (included in $W_{PBV}$) such that:

$$\sigma\left(W_{PBV} C_{fp}\right) = 1 \qquad\qquad (4)$$

Variations of the above may be useful too. Instead of choosing the gain so as to keep the standard deviation of the pulse signal constant, the amplitude peak in the FFT-domain of the pulse signal may be kept constant. Also, it is possible to keep a fixed ratio between the energy of the pulse signal (or just the energy of its fundamental frequency) and the energy in the output respiration signal frequency range.

In the described embodiments, so far, the "PBV-method" as described e.g. in the above cited paper of G. de Haan and A. van Leest, "Improved motion robustness of remote-PPG by using the blood volume pulse signature", as a basis for the computations. In further embodiments it is possible to use alternatives to $W_{PBV}$. It is equally possible to use any of the other methods mentioned in this paper to compute the weights used to combine the color channels to a vital sign signal with minimal distortions. Particularly, a good solution also results when using the chrominance based method, "CHRO", but also the "guided BSS-based methods" and even the older BSS-based methods, using periodicity of the pulse signal for component selection, provide viable options. Generally, the weights are calculated from the color signals filtered to include at least the pulse signal variations, while the respiration signal is derived from the color signals using the same weights, but a different filtering. Also the gain control can be derived from the standard deviation of the pulse signal, regardless the initial method used to derive the weighing vector.

Hence, in view of the above explained possible variations, said weight computation unit 34 may be configured to compute the weights w such that

- the weighted combination has a covariance with the individual detection signals that corresponds, as closely as possible, to a predefined vector,
- intensity variations and specular reflections are suppressed, or
- a demixing matrix is computed to identify independent signals in the detection channels and a vital sign signal is chosen from the independent signals using a second criterion.

In another preferred embodiment, the available skin area is divided into sub-regions and the aforementioned processing is performed per sub-region. This process leads to multiple candidate signals, $S_1$ and $S_2$, which can be directly combined into a final respiration signal (when only combining $S_2$), or from which the respiration signal can be derived by filtering (when combining $S_1$). The combining process may be a median, or trimmed-mean filtering, rejecting outliers, or can be a weighted average with weights e.g. determined by the variance, in the time domain, of the individual signals (this assumes that a high variance implies a high residual distortion).

Hence, in this embodiment the weight computation unit 34 computes weights w per set of at least two detection signals C acquired from different sub-regions. The vital sign signal computation unit 36 then computes, per set of at least two detection signals C, a first preliminary vital sign signal by a weighted combination of said at least two first bandwidth-limited detection signals $C_{f1}$ using the computed weights w of the respective set of at least two detection signals C and to compute said first vital sign signal $S_1$ by combining said first preliminary vital sign signal. Finally, the second filter unit 37 filters said first vital sign signal $S_1$ with a second filter to obtain said second vital sign signal $S_2$.

The general concept of combining signals from sub-regions has e.g. been described, including the various options, in WO 2014/024104 A1. A further alternative to find the weights to combine the signals from the sub-regions uses PCA or ICA, as e.g. described in W. Wang, S. Stuijk, and G. de Haan, "Exploiting Spatial-redundancy of Image Sensor for Motion Robust rPPG", IEEE, Tr. On Biomedical Engineering, 2014.

In a still further embodiment, the weights to minimize distortions are calculated for both the individual sub-regions and the entire skin area. These weights are consequently used to extract signals from these (sub-) regions including both respiration and pulse information. Selecting the best weights is accomplished by calculating the signal-to-noise ratio (SNR) of each region. By ranking the signals based on their SNRs, the final weights are selected, as either the weights corresponding to the signal with the highest SNR, or a combination of the weights corresponding to the signals (two or more) with the highest SNR. These final weights are then applied to the filtered, normalized traces of the entire skin region, containing only respiration information.

The above described embodiments have mainly been explained with respect to contactless sensors. Generally, the same methods can also be used for contact sensors. By way of example, the present invention can be applied in the field of health care, e.g. unobtrusive remote patient monitoring, general surveillances, security monitoring and so-called lifestyle environments, such as fitness equipment, or the like. Applications may include monitoring of oxygen saturation (pulse oximetry), heart rate, blood pressure, cardiac output, respiration, Mayer waves, changes of blood perfusion, assessment of autonomic functions, and detection of peripheral vascular diseases. The present invention can e.g. be used for rapid and reliable respiration monitoring and detection of a critical patient. The system can be used for monitoring of vital signs of neonates as well. In summary, the present invention improves the SNR considerably for near stationary subjects and consequently leads to a more accurate beat-to-beat measurement.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, which is defined by the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device for determining vital sign information of a subject, said device comprising:

   - an input interface (30) for obtaining at least two detection signals (C) derived from detected electromagnetic radiation transmitted through or reflected from a skin region of a subject, wherein each detection signal comprises wavelength-dependent reflection or transmission information in a different wavelength channel,
   - a first filter unit (32) for filtering said at least two detection signals (C) with a first filter to obtain at least two first bandwidth-limited detection signals ($C_{f1}$),
   - a weight computation unit (34) for computing weights (w) resulting, when applied in a weighted combination of said at least two first bandwidth-limited detection signals ($C_{f1}$), in a first vital sign signal ($S_1$) having reduced distortions,
   - a vital sign signal computation unit (36) for computing a second vital sign signal ($S_2$) different from the first vital sign signal ($S_1$) by using
   - either said first bandwidth-limited detection signals ($C_{f1}$), if they include the frequency range of said second vital sign signal ($S_2$), to compute the first vital sign signal ($S_1$) as a weighted combination of said at least two first bandwidth-limited detection signals ($C_{f1}$) using the computed weights (w) and to filter the computed first vital sign signal ($S_1$) with a second filter unit (37) of the device to obtain the second vital sign signal ($S_2$),
   - or, otherwise, a weighted combination of at least two second bandwidth-limited detection signals ($C_{f2}$) using said computed weights (w), the second bandwidth-limited detection signals obtained by filtering said at least two detection signals (C) with a further second filter unit (33) of the device, being differently bandwidth-limited than said first bandwidth-limited detection signals ($C_{f1}$) and including the frequency range of said second vital sign signal ($S_2$), and
   - a vital sign determination unit (38) for determining vital sign information from said second vital sign signal ($S_2$).

2. Device as claimed in claim 1,
wherein said first filter unit (32) is configured to let at least the frequency range of a subject's pulse rate pass and suppress a DC component.

3. Device as claimed in claim 2,
wherein said first filter unit (32) is configured to additionally let the frequency range of a subject's respiration signal and/or Mayer waves pass.

4. Device as claimed in claim 1,
wherein said weight computation unit (34) is configured to compute the weights (w) such that

- the weighted combination has a covariance with the individual detection signals that corresponds, as closely as possible, to a predefined vector,
- intensity variations and specular reflections are suppressed, or
- a demixing matrix is computed to identify independent signals in the detection channels and a vital sign signal is chosen from the independent signals using a second criterion.

5. Device as claimed in claim 1,
wherein said vital sign signal computation unit (36) is configured to compute the second vital sign signal ($S_2$) by a weighted combination of the at least two second bandwidth-limited detection signals ($C_{f2}$) using the computed weights (w).

6. Device as claimed in claim 1, wherein said second filter unit (37) is configured to let at least the frequency range of a subject's respiration signal and/or Mayer waves pass and suppress at least the frequency range of a subject's pulse signal.

7. Device as claimed in claim 1,
wherein said vital sign signal computation unit (36) is configured to compute a first vital sign signal ($S_1$) by a weighted combination of said at least two first bandwidth-limited detection signals ($C_{f1}$) using the computed weights (w), and wherein the device further comprises a characteristics detector (40) for detection of a characteristic of said first vital sign signal ($S_1$), in particular for peak detection in a frequency domain representation and/or amplitude or standard deviation detection in a time domain representation of said first vital sign signal ($S_1$), to obtain a gain (G), and a multiplication unit (42) for multiplying the second vital sign signal ($S_2$) with said gain (G).

8. Device as claimed in claim 1,
wherein the device is configured to compute a number of second vital sign signals ($S_2$), each from a different set of at least two detection signals (C) derived from detected electromagnetic radiation transmitted through or reflected from different skin regions of the subject, and wherein said vital sign determination unit (38) is configured to determine the vital sign information from a combination of said number of second vital sign signals ($S_2$).

9. Device as claimed in claim 1,
wherein said input interface (30) is configured to obtain different sets of at least two detection signals (C) derived from detected electromagnetic radiation transmitted through or reflected from different skin regions of the subject, wherein said weight computation unit (34) is configured to compute weights (w) per set of at least two detection signals (C), wherein said vital sign signal computation unit (36) is configured to compute, per set of at least two detection signals (C), a first preliminary vital sign signal by a weighted combination of said at least two first bandwidth-limited detection signals ($C_{f1}$) using the computed weights (w) of the respective set of at least two detection signals (C) and to compute said first vital sign signal ($S_1$) by combining said first preliminary vital sign signals computed for the different sets of at least two detection signals (C).

10. Device as claimed in claim 1,
wherein said weight computation unit (34) is configured to compute said weights (w) by setting a gain, used in the computation, such that the amplitude of said first vital sign signal ($S_1$) or of the standard deviation of said first vital sign signal ($S_1$) or of a characteristic, in particular a peak or a RMS-value of a small frequency range (around a peak), in the frequency domain representation of said first vital sign signal ($S_1$) is constant over time.

11. System for determining vital sign information of a subject, said system comprising:

- a detector (18, 19) for detecting electromagnetic radiation transmitted through or reflected from a skin region of a subject and for deriving at least two detection signals (C) from the detected electromagnetic radiation, wherein each detection signal comprises wavelength-dependent reflection or transmission information in a different wavelength channel,
- a device (12, 12a, 12b, 12c) as claimed in claim 1 for determining respiration information from said derived at least two detection signals (C).

12. Method for determining vital sign information of a subject by a computer or a device as claimed in claim 1, said method comprising:

- obtaining at least two detection signals (C) derived from detected electromagnetic radiation transmitted through or reflected from a skin region of a subject, wherein each detection signal comprises wavelength-dependent reflection or transmission information in a different wavelength channel,
- filtering said at least two detection signals (C) with a first filter to obtain at least two first bandwidth-limited detection signals ($C_{f1}$),
- computing weights (w) resulting, when applied in a weighted combination of said at least two first bandwidth-limited detection signals ($C_{f1}$), in a first vital sign signal ($S_1$) having reduced distortions,
- computing a second vital sign signal ($S_2$) different from the first vital sign signal ($S_1$) by using

- either said first bandwidth-limited detection signals ($C_{f1}$), if they include the frequency range of said second vital sign signal ($S_2$), to compute the first vital sign signal ($S_1$) as a weighted combination of said at least two first bandwidth-limited detection signals ($C_{f1}$) using the computed weights (w) and to filter the computed first vital sign signal ($S_1$) with a second filter unit (37) to obtain the second vital sign signal ($S_2$),
- or, otherwise, a weighted combination of at least two second bandwidth-limited detection signals ($C_{f2}$) using the computed weights (w), the second bandwidth-limited detection signals obtained by filtering said at least two detection signals (C) with a further second filter unit (33), being differently bandwidth-limited than said first bandwidth-limited detection signals ($C_{f1}$) and including the frequency range of said second vital sign signal ($S_2$), and

- determining vital sign information from said second vital sign signal ($S_2$).

13. Computer program comprising program code means for causing a computer to carry out the steps of the method as claimed in claim 12 when said computer program is carried out on the computer.

**Patentansprüche**

1. Vorrichtung zur Bestimmung der Vitalzeicheninformationen eines Patienten, wobei die genannte Vorrichtung Folgendes umfasst:

- eine Eingabeschnittstelle (30) zum Erlangen von mindestens zwei Detektionssignalen (C), die von detektierter elektromagnetischer Strahlung abgeleitet wurden, welche durch eine Hautregion eines Patienten gesendet oder von dieser reflektiert wurde, wobei jedes Detektionssignal wellenlängenabhängige Reflexions- oder Transmissionsinformationen in einem unterschiedlichen Wellenlängenkanal umfasst,
- eine erste Filtereinheit (32) zum Filtern der genannten mindestens zwei Detektionssignale (C) mit einem ersten Filter, um mindestens zwei erste bandbreitenbegrenzte Detektionssignale ($C_{f1}$) zu erlangen,
- eine Gewichtberechnungseinheit (34) zum Berechnen von Gewichten (w), die bei Anwendung in einer gewichteten Kombination der genannten mindestens zwei ersten bandbreitenbegrenzten Detektionssignale ($C_{f1}$) zu einem ersten Vitalzeichensignal ($S_1$) mit reduzierten Verzerrungen führen,
- eine Vitalzeichensignalberechnungseinheit (36) zum Berechnen eines zweiten Vitalzeichensignals ($S_2$), das sich von dem ersten Vitalzeichensignal ($S_1$) unterscheidet, durch Verwenden von

- entweder den genannten ersten bandbreitenbegrenzten Detektionssignalen ($C_{f1}$), wenn sie den Frequenzbereich des genannten zweiten Vitalzeichensignals ($S_2$) einschließen, um das erste Vitalzeichensignal ($S_1$) als eine gewichtete Kombination der genannten mindestens zwei ersten bandbreitenbegrenzten Detektionssignale ($C_{f1}$) unter Verwendung der berechneten Gewichte (w) zu berechnen und um das berechnete erste Vitalzeichensignal ($S_1$) mit einer zweiten Filtereinheit (37) der Vorrichtung zu filtern, um das zweite Vitalzeichensignal ($S_2$) zu erlangen,

- oder ansonsten einer gewichteten Kombination von mindestens zwei zweiten bandbreitenbegrenzten Detektionssignalen ($C_{f2}$) mit Verwendung der genannten berechneten Gewichte (w), wobei die zweiten bandbreitenbegrenzten Detektionssignale, die durch Filtern der genannten mindestens zwei Detektionssignale (C) mit einer weiteren zweiten Filtereinheit (33) der Vorrichtung erlangt wurden, anders bandbreitenbegrenzt sind als die genannten ersten bandbreitenbegrenzten Detektionssignale ($C_{f1}$) und den Frequenzbereich des genannten zweiten Vitalzeichensignals ($S_2$) einschließen, und

- eine Vitalzeichenbestimmungseinheit (38) zum Bestimmen von Vitalzeicheninformationen anhand des genannten zweiten Vitalzeichensignals ($S_2$).

2. Vorrichtung nach Anspruch 1,
wobei die genannte erste Filtereinheit (32) konfiguriert ist, um mindestens den Frequenzbereich einer Pulsfrequenz des Patienten durchzulassen und eine DC-Komponente zu unterdrücken.

3. Vorrichtung nach Anspruch 2,
wobei die genannte erste Filtereinheit (32) konfiguriert ist, um zusätzlich den Frequenzbereich eines Atmungssignals des Patienten und/oder von Mayer-Wellen durchzulassen.

4. Vorrichtung nach Anspruch 1,
wobei die genannte Gewichtberechnungseinheit (34) konfiguriert ist, um die Gewichte (w) derartig zu berechnen, dass

- die gewichtete Kombination eine Kovarianz mit den einzelnen Detektionssignalen hat, die so eng wie möglich einem vordefinierten Vektor entspricht,
- Intensitätsschwankungen und Spiegelreflexionen unterdrückt werden, oder
- eine Entmischungsmatrix berechnet wird, um unabhängige Signale in den Detektionskanälen zu identifizieren, und ein Vitalzeichensignal unter Verwendung eines zweiten Kriteriums aus den unabhängigen Signalen ausgewählt wird.

5. Vorrichtung nach Anspruch 1,
wobei die genannte Vitalzeichenberechnungseinheit (36) konfiguriert ist, um das zweite Vitalzeichensignal ($S_2$) durch eine gewichtete Kombination der mindestens zwei zweiten bandbreitenbegrenzten Detektionssignale ($C_{f2}$) unter Verwendung der berechneten Gewichte (w) zu berechnen.

6. Vorrichtung nach Anspruch 1,
wobei die genannte zweite Filtereinheit (37) konfiguriert ist, um mindestens den Frequenzbereich eines Atmungssignals des Patienten und/oder von Mayer-Wellen durchzulassen und mindestens den Frequenzbereich eines Pulssignals des Patienten zu unterdrücken.

7. Vorrichtung nach Anspruch 1,
wobei die genannte Vitalzeichensignalberechnungseinheit (36) konfiguriert ist, um ein erstes Vitalzeichensignal ($S_1$) durch eine gewichtete Kombination der genannten mindestens zwei ersten bandbreitenbegrenzten Detektionssignale ($C_{f1}$) unter Verwendung der berechneten Gewichte (w) zu berechnen, und wobei die Vorrichtung ferner einen Eigenschaftendetektor (40) zur Detektion einer Eigenschaft des genannten ersten Vitalzeichensignals ($S_1$), insbesondere zur Peak-Detektion in einer Frequenzdomänendarstellung und/oder Amplituden- oder Standardabweichungsdetektion in einer Zeitdomänendarstellung des genannten ersten Vitalzeichensignals ($S_1$), um eine Verstärkung (G) zu erlangen, und eine Multiplikationseinheit (42) zum Multiplizieren des zweiten Vitalzeichensignals ($S_2$) mit der genannten Verstärkung (G) umfasst.

8. Vorrichtung nach Anspruch 1,
wobei die Vorrichtung konfiguriert ist, um eine Anzahl von zweiten Vitalzeichensignalen ($S_2$) zu berechnen, jedes aus einer anderen Gruppe von mindestens zwei Detektionssignalen (C), die von detektierter elektromagnetischer Strahlung abgeleitet wurden, welche durch unterschiedliche Hautregionen des Patienten gesendet oder von diesen reflektiert wurde, und wobei die genannte Vitalzeichenbestimmungseinheit (38) konfiguriert ist, um die Vitalzeicheninformationen anhand einer Kombination der genannten Anzahl von zweiten Vitalzeichensignalen ($S_2$) zu bestimmen.

9. Vorrichtung nach Anspruch 1,

wobei die genannte Eingabeschnittstelle (30) konfiguriert ist, um verschiedene Gruppen von mindestens zwei Detektionssignalen (C) zu erlangen, die von detektierter elektromagnetischer Strahlung abgeleitet wurden, welche durch unterschiedliche Hautregionen des Patienten gesendet oder von diesen reflektiert wurde,

wobei die genannte Gewichtberechnungseinheit (34) konfiguriert ist, um Gewichte (w) pro Gruppe von mindestens zwei Detektionssignalen (C) zu berechnen,

wobei die genannte Vitalzeichensignalberechnungseinheit (36) konfiguriert ist, um pro Gruppe von mindestens zwei Detektionssignalen (C) ein erstes vorläufiges Vitalzeichensignal durch eine gewichtete Kombination der genannten mindestens zwei ersten bandbreitenbegrenzten Detektionssignale ($C_{f1}$) unter Verwendung der berechneten Gewichte (w) der jeweiligen Gruppe von mindestens zwei Detektionssignalen (C) zu berechnen und um das genannte erste Vitalzeichensignal ($S_1$) durch Kombinieren der genannten ersten vorläufigen Vitalzeichensignale zu berechnen, die für die unterschiedlichen Gruppen von mindestens zwei Detektionssignalen (C) berechnet wurden.

**10.** Vorrichtung nach Anspruch 1,

wobei die genannte Gewichtberechnungseinheit (34) konfiguriert ist, um die genannten Gewichte (w) zu berechnen, indem eine in der Berechnung verwendete Verstärkung derartig eingestellt wird, dass die Amplitude des genannten ersten Vitalzeichensignals ($S_1$) oder der Standardabweichung des genannten ersten Vitalzeichensignals ($S_1$) oder einer Eigenschaft, insbesondere ein Peak oder ein Effektivwert eines kleinen Frequenzbereichs (um einen Peak herum), in der Frequenzdomänendarstellung des genannten ersten Vitalzeichensignals ($S_1$) über die Zeit konstant ist.

**11.** System zur Bestimmung von Vitalzeicheninformationen eines Patienten, wobei das genannte System Folgendes umfasst:

- einen Detektor (18, 19) zum Detektieren von elektromagnetischer Strahlung, die durch eine Hautregion eines Patienten gesendet oder von dieser reflektiert wurde, und zum Ableiten von mindestens zwei Detektionssignalen (C) von der detektierten elektromagnetischen Strahlung, wobei jedes Detektionssignal wellenlängenabhängige Reflexions- oder Transmissionsinformationen in einem unterschiedlichen Wellenlängenkanal umfasst,
- eine Vorrichtung (12, 12a, 12b, 12c) nach Anspruch 1 zur Bestimmung von Atmungsinformationen anhand der genannten abgeleiteten mindestens zwei Detektionssignale (C).

**12.** Verfahren zur Bestimmung der Vitalzeicheninformationen eines Patienten durch einen Computer oder eine Vorrichtung nach Anspruch 1, wobei das genannte Verfahren Folgendes umfasst:

- Erlangen von mindestens zwei Detektionssignalen (C), die von detektierter elektromagnetischer Strahlung abgeleitet wurden, welche durch eine Hautregion eines Patienten gesendet oder von dieser reflektiert wurde, wobei jedes Detektionssignal wellenlängenabhängige Reflexions- oder Transmissionsinformationen in einem unterschiedlichen Wellenlängenkanal umfasst,
- Filtern der genannten mindestens zwei Detektionssignale (C) mit einem ersten Filter, um mindestens zwei erste bandbreitenbegrenzte Detektionssignale ($C_{f1}$) zu erlangen,
- Berechnen von Gewichten (w), die bei Anwendung in einer gewichteten Kombination der genannten mindestens zwei ersten bandbreitenbegrenzten Detektionssignale ($C_{f1}$) zu einem ersten Vitalzeichensignal ($S_1$) mit reduzierten Verzerrungen führen,
- Berechnen eines zweiten Vitalzeichensignals ($S_2$), das sich von dem ersten Vitalzeichensignal ($S_1$) unterscheidet, durch Verwenden von

- entweder den genannten ersten bandbreitenbegrenzten Detektionssignalen ($C_{f1}$), wenn sie den Frequenzbereich des genannten zweiten Vitalzeichensignals ($S_2$) einschließen, um das erste Vitalzeichensignal ($S_1$) als eine gewichtete Kombination der genannten mindestens zwei ersten bandbreitenbegrenzten Detektionssignale ($C_{f1}$) unter Verwendung der berechneten Gewichte (w) zu berechnen und um das berechnete erste Vitalzeichensignal ($S_1$) mit einer zweiten Filtereinheit (37) zu filtern, um das zweite Vitalzeichensignal ($S_2$) zu erlangen,
- oder ansonsten einer gewichteten Kombination von mindestens zwei zweiten bandbreitenbegrenzten Detektionssignalen ($C_{f2}$) mit Verwendung der genannten berechneten Gewichte (w), wobei die zweiten bandbreitenbegrenzten Detektionssignale, die durch Filtern der genannten mindestens zwei Detektionssignale (C) mit einer weiteren zweiten Filtereinheit (33) erlangt wurden, anders bandbreitenbegrenzt sind als die genannten ersten bandbreitenbegrenzten Detektionssignale ($C_{f1}$) und den Frequenzbereich des genannten zweiten Vitalzeichensignals ($S_2$) einschließen, und

- Bestimmen von Vitalzeicheninformationen anhand des genannten zweiten Vitalzeichensignals ($S_2$).

**13.** Computerprogramm umfassend Programmcodemittel zum Veranlassen eines Computers zur Durchführung der Schritte des Verfahrens nach Anspruch 12, wenn das genannte Computerprogramm auf dem Computer ausgeführt wird.

**Revendications**

**1.** Dispositif de détermination d'informations de signes vitaux d'un sujet, ledit dispositif comprenant :

- une interface d'entrée (30) pour obtenir au moins deux signaux de détection (C) dérivés d'un rayonnement électromagnétique détecté transmis à travers ou réfléchi depuis une région de peau d'un sujet, dans lequel chaque signal de détection comprend des informations de réflexion ou de transmission dépendant de la longueur d'onde dans un canal de longueur d'onde différent,
- une première unité de filtre (32) pour filtrer lesdits au moins deux signaux de détection (C) avec un premier filtre pour obtenir au moins deux premiers signaux de détection limités en largeur de bande ($C_{f1}$),
- une unité de calcul de poids (34) pour calculer des poids (w) donnant, lorsqu'ils sont appliqués dans une combinaison pondérée desdits au moins deux premiers signaux de détection limités en largeur de bande ($C_{f1}$), un premier signal de signes vitaux ($S_1$) ayant des distorsions réduites,
- une unité de calcul de signal de signes vitaux (36) pour calculer un second signal de signes vitaux ($S_2$) différent du premier signal de signes vitaux ($S_1$) en utilisant
- soit lesdits premiers signaux de détection limités en largeur de bande ($C_{f1}$), s'ils incluent la plage de fréquence dudit second signal de signes vitaux ($S_2$), pour calculer le premier signal de signes vitaux ($S_1$) en tant que combinaison pondérée desdits au moins deux premiers signaux de détection limités en largeur de bande ($C_{f1}$) à l'aide des poids calculés (w) et pour filtrer le premier signal de signes vitaux ($S_1$) calculé avec une seconde unité de filtre (37) du dispositif pour obtenir le second signal de signes vitaux ($S_2$),
- soit sinon, une combinaison pondérée d'au moins deux seconds signaux de détection limités en largeur de bande ($C_{f2}$) à l'aide desdits poids calculés (w), les seconds signaux de détection limités en largeur de bande obtenus par filtrage desdits au moins deux signaux de détection (C) avec une seconde unité de filtre supplémentaire (33) du dispositif, étant limités en largeur de bande différemment desdits premiers signaux de détection limités en largeur de bande ($C_{f1}$) et incluant la plage de fréquence dudit second signal de signes vitaux ($S_2$), et
- une unité de détermination de signes vitaux (38) pour déterminer des informations de signes vitaux à partir dudit second signal de signes vitaux ($S_2$).

**2.** Dispositif selon la revendication 1,
dans lequel ladite première unité de filtre (32) est configurée pour laisser passer au moins la plage de fréquence d'une fréquence de pouls d'un sujet et supprimer une composante CC.

**3.** Dispositif selon la revendication 2,
dans lequel ladite première unité de filtre (32) est configurée pour laisser passer en outre la plage de fréquence d'un signal de respiration d'un sujet et/ou des ondes Mayer.

**4.** Dispositif selon la revendication 1,
dans lequel ladite unité de calcul de poids (34) est configurée pour calculer les poids (w) de sorte que

- la combinaison pondérée ait une covariance avec les signaux de détection individuels qui correspond, aussi étroitement que possible, à un vecteur prédéfini,
- des variations d'intensité et des réflexions spéculaires soient supprimées, ou
- une matrice de démixage soit calculée pour identifier des signaux indépendants dans les canaux de détection et un signal de signes vitaux soit choisi parmi les signaux indépendants à l'aide d'un second critère.

**5.** Dispositif selon la revendication 1,
dans lequel ladite unité de calcul de signal de signes vitaux (36) est configurée pour calculer le second signal de signes vitaux ($S_2$) par une combinaison pondérée des au moins deux seconds signaux de détection limités en largeur de bande ($C_{f2}$) à l'aide des poids calculés (w).

**6.** Dispositif selon la revendication 1,

dans lequel ladite seconde unité de filtre (37) est configurée pour laisser passer au moins la plage de fréquence d'un signal de respiration d'un sujet et/ou des ondes Mayer et supprimer au moins la plage de fréquence d'un signal de pouls d'un sujet.

7. Dispositif selon la revendication 1,
dans lequel ladite unité de calcul de signal de signes vitaux (36) est configurée pour calculer un premier signal de signes vitaux ($S_1$) par une combinaison pondérée desdits au moins deux premiers signaux de détection limités en largeur de bande ($C_{f1}$) à l'aide des poids calculés (w), et
dans lequel le dispositif comprend en outre un détecteur de caractéristique (40) pour détecter une caractéristique dudit premier signal de signes vitaux ($S_1$), en particulier pour détecter une crête dans une représentation de domaine de fréquence et/ou détecter une amplitude ou un écart-type dans une représentation de domaine de temps dudit premier signal de signes vitaux ($S_1$), pour obtenir un gain (G), et une unité de multiplication (42) pour multiplier le second signal de signes vitaux ($S_2$) par ledit gain (G).

8. Dispositif selon la revendication 1,
dans lequel le dispositif est configuré pour calculer un nombre de seconds signaux de signes vitaux ($S_2$), chacun provenant d'un ensemble différent d'au moins deux signaux de détection (C) dérivés d'un rayonnement électromagnétique détecté transmis à travers ou réfléchi depuis des régions de peau différentes du sujet, et dans lequel ladite unité de détermination de signes vitaux (38) est configurée pour déterminer les informations de signes vitaux à partir d'une combinaison dudit nombre de seconds signaux de signes vitaux ($S_2$).

9. Dispositif selon la revendication 1,
dans lequel ladite interface d'entrée (30) est configurée pour obtenir des ensembles différents d'au moins deux signaux de détection (C) dérivés d'un rayonnement électromagnétique détecté transmis à travers ou réfléchi depuis des régions de peau différentes du sujet,
dans lequel ladite unité de calcul de poids (34) est configurée pour calculer des poids (w) par ensemble d'au moins deux signaux de détection (C),
dans lequel ladite unité de calcul de signal de signes vitaux (36) est configurée pour calculer, par ensemble d'au moins deux signaux de détection (C), un premier signal de signes vitaux préliminaire par une combinaison pondérée desdits au moins deux premiers signaux de détection limités en largeur de bande ($C_{f1}$) à l'aide des poids calculés (w) de l'ensemble respectif d'au moins deux signaux de détection (C) et pour calculer ledit premier signal de signes vitaux ($S_1$) en combinant lesdits premiers signaux de signes vitaux préliminaires calculés pour les ensembles différents d'au moins deux signaux de détection (C).

10. Dispositif selon la revendication 1,
dans lequel ladite unité de calcul de poids (34) est configurée pour calculer lesdits poids (w) en fixant un gain, utilisé dans le calcul, de sorte que l'amplitude dudit premier signal de signes vitaux ($S_1$) ou de l'écart-type dudit premier signal de signes vitaux ($S_1$) ou d'une caractéristique, en particulier d'une crête ou d'une valeur efficace d'une petite plage de fréquence (autour d'une crête), dans la représentation de domaine de fréquence dudit premier signal de signes vitaux ($S_1$) soit constante dans le temps.

11. Système de détermination d'informations de signes vitaux d'un sujet, ledit système comprenant :

- un détecteur (18, 19) pour détecter un rayonnement électromagnétique transmis à travers ou réfléchi depuis une région de peau d'un sujet et pour dériver au moins deux signaux de détection (C) du rayonnement électromagnétique détecté, dans lequel chaque signal de détection comprend des informations de réflexion ou de transmission dépendant de la longueur d'onde dans un canal de longueur d'onde différent,
- un dispositif (12, 12a, 12b, 12c) tel que revendiqué à la revendication 1 pour déterminer des informations de respiration à partir desdits au moins deux signaux de détection (C) dérivés.

12. Procédé de détermination d'informations de signes vitaux d'un sujet par un ordinateur ou un dispositif tel que revendiqué à la revendication 1, ledit procédé comprenant :

- l'obtention d'au moins deux signaux de détection (C) dérivés d'un rayonnement électromagnétique détecté transmis à travers ou réfléchi depuis une région de peau d'un sujet, dans lequel chaque signal de détection comprend des informations de réflexion ou de transmission dépendant de la longueur d'onde dans un canal de longueur d'onde différent,
- le filtrage desdits au moins deux signaux de détection (C) avec un premier filtre pour obtenir au moins deux premiers signaux de détection limités en largeur de bande ($C_{f1}$),
- le calcul de poids (w) donnant, lorsqu'ils sont appliqués dans une combinaison pondérée desdits au moins deux premiers signaux de détection limités en largeur de bande ($C_{f1}$), un premier signal de signes vitaux ($S_1$) ayant des distorsions réduites,
- le calcul d'un second signal de signes vitaux ($S_2$) différent du premier signal de signes vitaux ($S_1$) en utilisant
- soit lesdits premiers signaux de détection limités en largeur de bande ($C_{f1}$), s'ils incluent la plage de fréquence dudit second signal de signes vitaux ($S_2$), pour calculer le premier signal de signes vitaux ($S_1$) en tant que combinaison pondérée desdits au moins deux premiers signaux de détection limités en largeur de bande ($C_{f1}$) à l'aide des poids calculés (w) et pour filtrer le premier signal de signes vitaux calculé ($S_1$) avec une seconde unité de filtre (37) pour obtenir le second signal de signes vitaux ($S_2$),
- soit sinon, une combinaison pondérée d'au moins deux seconds signaux de détection limités en largeur de bande ($C_{f2}$) à l'aide desdits poids calculés (w), les seconds signaux de détection limités en largeur de bande obtenus par filtrage desdits au moins deux signaux de détection (C) avec une seconde unité de filtre supplémentaire (33), étant limités en largeur de bande différemment desdits premiers signaux de détection limités en largeur de bande ($C_{f1}$) et incluant la plage de fréquence dudit second signal de signes vitaux ($S_2$), et
- la détermination d'informations de signes vitaux à partir dudit second signal de signes vitaux ($S_2$).

**13.** Programme d'ordinateur comprenant un moyen de code de programme pour amener un ordinateur à réaliser les étapes du procédé tel que revendiqué à la revendication 12 lorsque ledit programme d'ordinateur est réalisé sur l'ordinateur.

FIG.1

FIG.2

FIG.3

FIG.4

FIG.5

## REFERENCES CITED IN THE DESCRIPTION

### Patent documents cited in the description

- US 20140275825 A1
- US 20150320363 A1
- US 2013271591 A1
- WO 2014024104 A1

### Non-patent literature cited in the description

- **VERKRUYSSE et al.** Remote plethysmographic imaging using ambient light. *Optics Express,* 22 December 2008, vol. 16 (26), 21434-21445
- **G. DE HAAN ; A. VAN LEEST.** Improved motion robustness of remote-PPG by using the blood volume pulse signature. *Physiol. Meas.,* 2014, vol. 35, 1913
- Motion-Resistant Remote Imaging Photoplethysmography Based on the Optical Properties of Skin. **FENG LITONG et al.** IEEE TRANSACTIONS ON CIRCUITS AND SYSTEMS FOR VIDEO TECHNOLOGY. IEEE SERVICE CENTER, 01 May 2015, vol. 25, 879-891
- **G. DE HAAN ; A. VAN LEEST.** Improved motion robustness of remote-PPG by using the blood volume pulse signature. *Physiol. Meas,* 2014, vol. 35, 1913
- Exploiting Spatial-redundancy of Image Sensor for Motion Robust rPPG. **W. WANG ; S. STUIJK ; G. DE HAAN.** Tr. On Biomedical Engineering. IEEE, 2014